# EUROPEAN PATENT APPLICATION

(11) **EP 3 117 841 A1**
(43) Date of publication of application: **18.01.2017**
(21) Application number: 15176988.2
(22) Date of filing: 16.07.2015
(51) Int. Cl.: A61L 9/03

(54) **AUTONOMOUSLY POWERED VAPOUR RELEASE DEVICE**

(71) Applicant: Eurvest, 1400 Nivelles (BE)
(72) Inventor: Luciani, Alain, 13390 Auriol (FR); Wos, Magdalena, 20-583 Lublin (PL); Bertignon, Estelle, 7000 Mons (BE); Dmitruk, Aleksandra, 20-461 Lublin (PL); Slawek, Monika, 20-553 Lublin (PL)
(74) Representative: August & Debouzy avocats

(57) **Abstract**

The present invention is directed to an autonomously powered vapor dispensing device comprising an intermediate basin (2) which allows the volatile liquid contained therein to impregnate a dispensing material by capillarity and dispense into the atmosphere; and a heating element (4) which heats the intermediate basin (2) comprising the volatile liquid to force evaporation cyclically of the volatile liquid directly from the intermediate basin.

## Description

### FIELD OF THE INVENTION

The present invention relates to an autonomously powered vapor dispensing device for the controlled release of volatile compositions. More particularly the instant invention deals with means of dispersing a fragrance into a closed atmosphere, typically a house.

### TECHNICAL BACKGROUND

Volatile compositions such as fragrances can be released into the atmosphere. Some devices release the composition by convection, or passive diffusion.

For example, it is known in the art to fill a closed basin with a volatile liquid. The basin comprises one slot to allow one rigid strip to dip into the volatile liquid. While one of the extremities of the strip is in contact with the liquid of the basin, the other one is in contact with the external environment. Typically, the composition travels along the strip by capillarity to be released in the environment.

These devices however comprise several drawbacks as their release capabilities is limited. For example, the strip is subjected to a clogging effect: the prolonged use of the dispensing material leads to an internal degradation of the material which eventually impacts its capability to release the composition into the environment. Therefore, it is usually necessary to change the dispensing material although the user is not completely certain of the best time to change it. The user further needs to touch the used material which leads to chemical contamination. Similar types of dispenser are disclosed in WO 1995/005855.

Other devices of the type "plug and smell" have also been disclosed in the art. These solutions involve a dispenser which is adapted to receive a supply of volatile liquid from a capsule or a reservoir that is plugged to the dispenser. This type of dispenser is disclosed for example in WO 2010/120961. However, the opening of the reservoir is provided in the form of a rupturable wall which is covered with a micro-porous layer which is part of the supply device. As such, the layer is not however a dispensing material as it only allows the progressive release of the liquid out of the reservoir into the dispenser.

Nevertheless, this type of solution still has the drawback of entertaining an uneven rate of dispersion of the volatile composition. As the amount of liquid contained in the reservoir decreases, the rate of dispersion also decreases. A similar drawback is observed with the basin dispensers.

Other dispensers are electrically powered to actively enhance the delivery of the volatile composition, i.e. by heating the composition contained in the reservoir.

However, these devices do not offer the same flexibility to the user compared to passive dispensers as he needs to plug the dispenser directly to the electrical outlet. Thus, there is no freedom of movement around the room. Furthermore, these vapor dispensing devices are usually intended to operate over an extended period of time and occupy valuable electrical outlets once in service.

Another drawback of the known active devices derives from the finding that some users prefer to hide dispenser, e.g. behind an object, while it remains noticeable on an electrical outlet.

More particularly, in the case of release of fragrance, any of the passive or electrical dispensers release the volatile liquid at a continuous rate which eventually leads to the habituation of the customer to the fragrance being released.

Thus, there is still a need for an autonomous dispenser which overcomes all the above mentioned drawbacks while achieving an increased release efficacy.

### SUMMARY OF THE INVENTION

This has been achieved with the instant invention.

A first aspect of the invention is concerned with an autonomously powered vapor dispensing device comprising an intermediate basin which allows the volatile liquid contained therein to impregnate a dispensing material by capillarity and dispense into the atmosphere; and a heating element which heats either the volatile liquid directly or heats the intermediate basin comprising the volatile liquid to force evaporation cyclically of the volatile liquid directly from the intermediate basin.

According to one embodiment of the invention, the dispensing device comprises an intermediate basin that is fed by a replaceable reservoir to be placed onto the intermediate basin and is closed by a rupturable substrate that opens upon placing on said basin.

According to one embodiment of the invention, the dispersing material is framed with a coupling element which holds together the reservoir and the dispensing material onto the basin, preferably in a vertically straight position.

According to one embodiment of the invention, the dispensing device has a level of volatile liquid contained into the basin maintained constant throughout the regular use of the device.

According to one embodiment of the invention, the dispensing device is adapted to release a volatile liquid from an impregnated dispensing material by passive convection and further adapted to cyclically heat the volatile liquid over a period of 10 seconds to 5 minutes, preferably 10 seconds to 2 minutes, more preferably 10 seconds to 1 minute to enhance said convection.

According to one embodiment of the invention, the dispensing device involves cycles that are repeated each 20 to 45 minutes, preferably each 30 minutes.

According to one embodiment of the invention, the dispensing device is programmed to automatically stop after the occurrence of a preset number of cycles.

According to one embodiment of the invention, the temperature of the volatile liquid of the dispensing device is heated by 15°C, preferably by 10°C and more preferably by 5°C above room temperature.

According to one embodiment of the invention, the dispensing device involves a constant temperature above room temperature constantly maintained over a predetermined period of time of more than 10 seconds and does not exceed 7 minutes, preferably does not exceed 4 minutes and more preferably does not exceed 2 minutes.

According to one embodiment of the invention, the dispensing device is powered by batteries.

According to one embodiment of the invention, the dispensing device involves batteries that are rechargeable by induction.

According to one embodiment of the invention, the dispensing device involves a heating element that corresponds to at least one light bulb or at least one resistor.

A second aspect of the invention is concerned with a process for the manufacture of the device, comprising the steps consisting in fixing a reservoir in the neck of the dispensing device dispensing in order to release the volatile liquid into the intermediate basin.

Another aspect of the invention is concerned with a use of a dispensing device to release a fragrance.

Another aspect of the invention is concerned with a use which further prevents the habituation of the fragrance to the user over the entire lifetime of the dispenser.

### FIGURES

Figure 1 a and 1 b illustrate a supply device together with a dispensing material that may be used in a dispenser according to the invention.
Figures 2a-c respectively illustrate the overall view of an appropriate basin that can be used in a dispenser according to the invention; a top view of said basin and its sectional B-B view.
Figure 3 represents a cross section of a dispenser according to the invention.
Figure 4 is an exploded representation of an assembled dispenser according to the invention.
Figures 5a and b respectively represent the cyclic heating of the heating element present in the instant invention, together with a detailed representation of one of the cycle, representing the cyclic active release of the fragrance.

### DESCRIPTION OF EMBODIMENTS OF THE INVENTION

The present invention relates to an autonomous apparatus or device for the delivery of a volatile liquid into the atmosphere.

Within the meaning of the present invention, the term "volatile liquid" means any liquid composition which evaporates at ambient temperature. The apparatus of the present invention is suitable for purposes of providing fragrances, air fresheners, deodorizers, odor eliminators, malodor counteractants, insecticides, insect repellants, medicinal substances, disinfectants, sanitizers, mood enhancers, and aromatherapy aids, or for any other purpose using a volatile material that acts to condition, modify, or otherwise change the atmosphere or the environment. Particularly, the device according to the invention is intended to provide autonomously fragrance to the environment.

The device according to the invention is useful for domestic use, e.g. to release autonomously a volatile composition into one or more room of a house, office or any other confined space such as a car or a cabin.

The continuous emission of the volatile materials with the devices of the invention can be of any suitable length, including but not limited to, up to: 20 days, 30 days, 60 days, 90 days, shorter or longer periods, or any period between 30 to 90 days. The duration of the emission will depend on the amount of liquid volatile supplied to the device. It can be adapted according to the contemplated use and/or location.

One essential aspect of the dispensers according to the invention is that they are autonomous, the term designating devices which are powered, at least temporarily, by an autonomous source of energy, rather than by being coupled to a central system such as an electric system in a house. Typically, an autonomous power element can be any of a battery system, solar cells and the like. The ability to move freely the dispenser from one place to another around the house while being used is thus preserved.

The dispensers according to the invention comprise different elements which all cooperate together to dispense the vapor into the environment.

These elements typically include a supply unit; an intermediate basin; a dispensing material and an autonomous power.

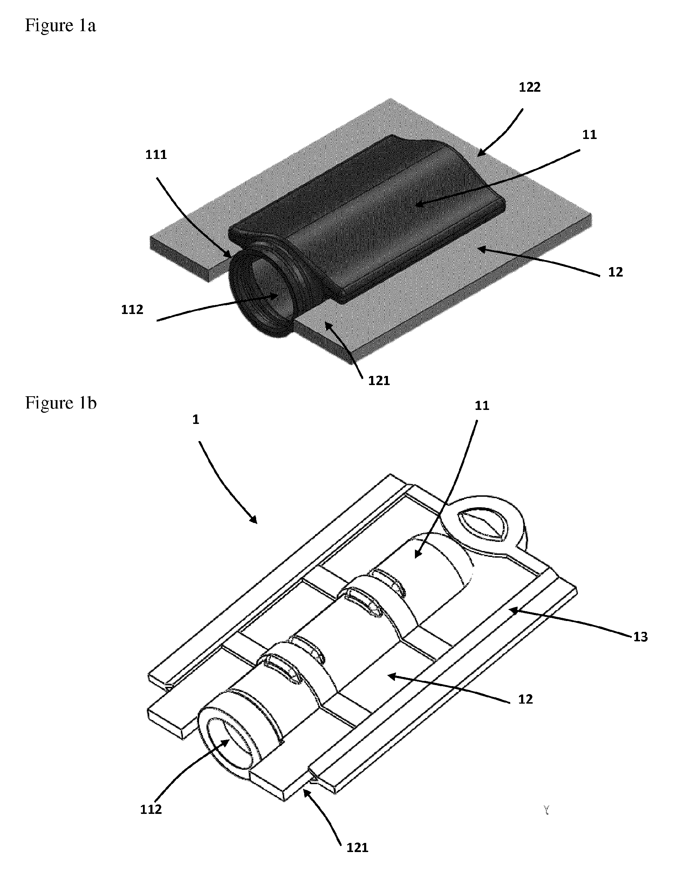

The supply unit comprises a reservoir. In reference to the content of Figures 1, the reservoir (11) corresponds to an impermeable recipient which is adapted to contain a volatile liquid. Appropriate reservoirs have a capacity comprised within the range of 3 to 100 ml, typically within the range of 6 to 60 ml, preferably within the range of 10 to 20 ml. The capability of the reservoir can be adapted to the time of dispersion of the vapor contemplated with the system and/or the size of the basin used with the supplying unit.

Typically, the reservoirs envisaged in the present invention comprise a neck (111) and one opening (112) which corresponds to either side of the reservoir or only a portion of it. Notably the reservoir corresponds to a bottle as it appears in the Figures 1. Such bottles are available in the art and easily manufactured which is convenient for the overall manufacture of the dispensing device. During storage the reservoir is closed to isolate the volatile liquid from the exterior atmosphere and prevent its vaporization. The reservoir is configured to open when it is adapted on the basin.

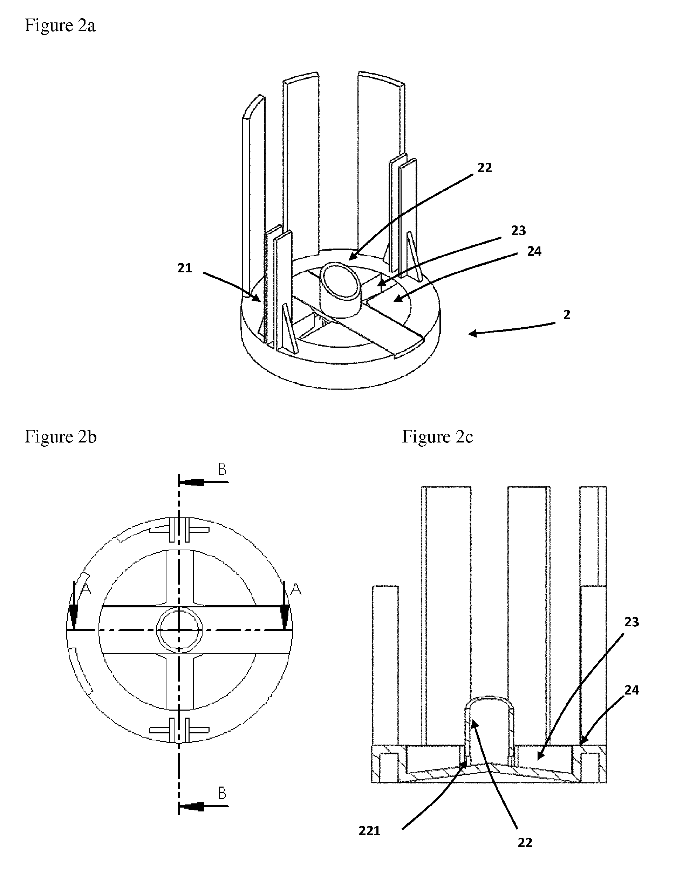

Advantageously, the reservoir has an opening (112) materialized in the form of a rupturable substrate. This type of opening is known and available in the art with various types of materials. It allows the reservoir to be safely handled without risk of spillage and only opens when the reservoir is inserted into the basin or placed on adapted means. Typically, the reservoir is placed with the opening section facing downwards onto the external extension of the neck of the basin, as illustrated in Figure 2a. The neck and opening have a size which fits the size and form of the opening of the reservoir and ruptures the substrate to release the volatile liquid into the basin while maintaining the reservoir into the same position. The basin is then supplied by gravity with the volatile liquid.

Another element of the instant dispensers is the intermediate basin. This element typically corresponds to a recipient which is adapted to contain a volatile liquid therein. The term "intermediate" indicates that it contains the volatile liquid to be dispersed by the dispensing material albeit supplied from the reservoir unit.

The basin may have different shapes, e.g. cubic, rectangular or circular. It is substantially closed to prevent the free dispersion of the volatile liquid but comprises at least one opening to let a dispensing element plunge into the volatile liquid; traverse the basin and come in contact with the surrounding atmosphere to dispense the vapor.

Appropriate basins are made of an impermeable material, for example of plastic, which are suited to contain a volatile liquid therein. Appropriate dimensions for a basin are: a length comprised within the range of 30 to 80 mm, typically within the range of 50 to 70 mm, a width comprised within the range of 5 to 15 mm, typically 9 to 12 mm; a thickness comprised within the range of 5 to 15 mm, typically within the range of 8 to 12 mm. These dimensions are appropriate to use the vapor dispensing device in different locations, e.g. in a house or other confined spaces. It does not have to be hanged and can advantageously be left on a piece of furniture.

In reference to the embodiment illustrated in Figures 2a the basin (2) is substantially closed except on the upper side (24) of the basin where a longitudinal hole (23) allows a dispensing strip to traverse the wall of the recipient. When the volatile liquid is contained inside the basin and the dispensing material plunged inside the basin, the volatile liquid rises by capillarity along the material of the dispensing strip until it gets in contact with the surrounding atmosphere where it is released. Typically, other shapes can be adopted for the hole of the basin provided that it allows the dispensing material to come in contact with the volatile liquid contained within the basin. As further exemplified in Figure 2b, the basin does not need to have the same depth over the entire section. In reference to the example illustrated in Figures 2a and 2b, the basin advantageously comprises a neck (22). This neck is located on the upper surface of the basin (24) and extends outside and inside the basin to come in contact with the reservoir of the supplying unit (not represented on the Figures 2). The neck leads the volatile liquid from the reservoir to the interior cavity of the basin (2). The inside extension of the neck is further illustrated in the sectional view of Figure 2b.

When a volatile liquid is supplied continuously to the basin, for example from a reservoir, it fills the basin and the level of liquid within the basin rises to the point where it reaches the lower part of the neck or the lower level of the reservoir. The level of liquid within the basin then remains constant and the amount of volatile liquid which is released into the atmosphere is constantly compensated by gravity from the reservoir until it eventually gets empty. A new reservoir is then replaced and adapted to the basin to supply a new amount of volatile liquid to the dispensing system. The basin considered within the present invention operates in a similar manner as a bird watering system.

Advantageously, the internal extension of the neck (22) of the basin comprises a slot which allows the air to enter more easily through the neck into the reservoir in order to maintain a continuous flow of liquid from the reservoir to the basin and helps to maintain a constant level of volatile liquid within the basin. Such slot is represented on the sectional view of Figure 2c with the reference (221).

This aspect of the invention is particularly advantageous in comparison to products which are deprived of fixed reservoir where the basin needs to be refilled with volatile liquid on a regular basis. When the amount of liquid contained within the basin decreases, the amount of liquid which remains in contact with the dispensing material also decreases which eventually affects its capillarity capability and affects the rate of release of the vapor to the atmosphere. The instant invention may overcome this problem by ensuring a constant rate of dispersion of vapor, since the level of liquid is kept constant.

The liquid contained in the intermediate reservoir is dispensed in the surrounding atmosphere by the dispensing material (121). This material is responsible for the release of the vapor into the atmosphere when it comes in contact with the liquid contained within the basin at the time the reservoir is adapted to the basin to supply the system with new volatile liquid.

Typically, a dispensing material is a material which exhibits a porosity and capillarity to allow a volatile liquid to impregnate and travel along its inner structure. Suitable materials encompass wood, ceramics, terracotta paper, cotton, synthetic fiber, porous plastic and the like, preferably synthetic fibers. Other materials may be envisaged within the scope of the invention.

The dispensing material may have different forms and sizes. It is adapted to enter in contact with the volatile liquid contained within the basin, for example by extending beyond the level materialized by the opening of the reservoir to plunge into the liquid of the basin. By way of example, this extension is materialized in Figures 1 as element (121). The dispensing material is not directly in contact with the liquid contained within the reservoir but rather gets in contact with the liquid via the basin of the dispensing device.

Advantageously, the dispensing material is intimately coupled to the reservoir but not the other elements of the dispenser such as the basin or surrounding cap. This association of means provides an advantageous way to balance the lifetime of the dispensing material with the amount of volatile liquid supplied to the system. When the reservoir needs to be replaced, a new and clean dispensing material comes with the new supplying unit.

This prevents clogging problems, usually encountered with the dispensing systems that use the same piece of material to disperse the vapor throughout the entire lifetime of the device or when the user cannot correctly assess when is the best time to replace the material.

According to one aspect of the invention, the dispensing material does not have to be directly in contact with the liquid contained within the reservoir, e.g. as part of a wall of the reservoir or as part of the opening element of the reservoir. This facilitates the manufacture of the supplying unit and increases the life expectancy of the dispensing material which is not soaked with the liquid before the supplying unit is actually operated. It further prevents the product from being activated before use during storage.

The size and overall surface of the dispensing material can be adapted to reach a specific rate of release of the vapor and adapted to a given use. Typically, the higher is the surface of dispensing material in contact with the atmosphere, the higher is the amount of vapor released to the environment. For example, an appropriate surface of exchange of dispensing material is comprised within the range of 2 000 to 10 000 mm², typically within the range of 3 000 to 7 000 mm² such as about 4 000 mm². A surface of exchange within the meaning of the present invention should be construed as the surface of the dispensing material outside the basin which is in contact with the atmosphere once the supplying unit is placed on the basin.

Typically when a strip extends radially from the reservoir, the surface of exchange corresponds to the surface of both sides which is in contact with the atmosphere. Advantageously, the product according to the present invention offers a wider surface of exchange in comparison to the other existing devices and thus achieves a higher deodorizing effect.

For example, the dispensing material is in the form of at least one strip which is coupled to the reservoir. When several strips are used, these are arranged radially from the outer surface of the reservoir, preferably as part of the surface which corresponds to the symmetry plan of the reservoir.

In reference to the embodiment illustrated in Figure 1a, the dispensing material is coupled on both sides of the reservoir, within the plan of symmetry of the specific form of said reservoir (11). The dispensing material further extends below the opening section of the reservoir (121) to be able to plunge below the level of liquid of the basin into the liquid and extends beyond it upper extremity (122) to increase the overall surface of exchange with the surrounding environment. According to another embodiment, the dispensing material does not extend only over the entire height of the reservoir but extends below its opening section. Another appropriate design corresponds to an asymmetric dispensing material, where several strips are coupled to the outer surface of the reservoir and connected between them while only one strip extends below the opening section to reach the liquid.

According to the instant invention, the dispensing material may further cover all the outer surface of the reservoir or may comprise leave one portion free to let the user size the reservoir with his fingers without any risk of chemical contamination and without touching the wet impregnated dispensing material, i.e. when the supplying unit is empty.

The dispensing material can be directly coupled to the outer surface of the reservoir, e.g. with glue or any other suitable means available in the art. The manufacturing process is thus easily implemented as it does not require a complex structure for the reservoir.

According to another embodiment, the material is coupled to the outer surface of the reservoir with an intermediate element which maintains together the two means. For example, as illustrated in Figure 1b, a frame (13) may hold together the strips of the dispensing material onto the outer surface of the reservoir. The term frame within the meaning of the invention encompasses a cage or any element which at least surrounds the periphery of the dispensing material.

The frame (13) may further be used as a guide to direct the supply unit towards a given location on the basin when it is placed on it. It also serves as a supporting mean to maintain the supply unit in a given position. Depending on the design of the reservoir and the position of its opening section, the frame keeps the supply unit either inclined or vertically straight, to ensure that the liquid contained in the reservoir keeps flowing inside the basin. A vertical position is preferred to prevent any loss of liquid inside the reservoir during use. The term vertically straight means a position which forms substantially a 90° angle with the basis of the basin.

Typically, the frame may cooperate and be inserted in a rail located on each side of the basin when it is placed on it. Other guiding means placed either on the basin or the frame of the supply unit can be envisaged within the instant invention such as a clip or a prominent element.

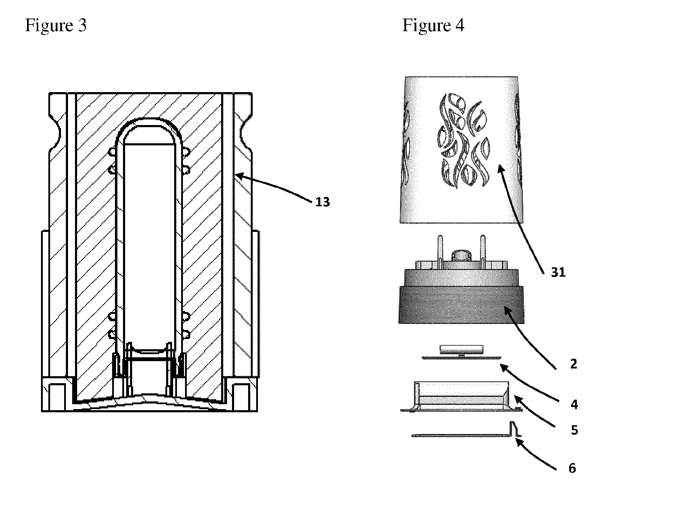

In reference to the embodiment illustrated in Figure 3, a frame element ensures the overall integrity of the strips and reservoir to provide a single unit which is directly handled by the user. The frame also serves as a guide to adapt the supply unit on the basin so that the opening section of the reservoir gets in line with the neck of the basin and remains straight during use. The Figure 3 further represents the two holes on the upper side of the basin which let the strips plunge into the volatile liquid contained within the cavity. As illustrated in Figure 3, the frame (13) of the supply unit is used in cooperation with a rail element (21) placed on each side of the basin. The frame is guided by the rail element and maintained in a vertically straight position onto the basin while bringing the dispensing material in contact with the volatile liquid contained within the basin (2) through the appropriate holes (23). As further illustrated in Figure 3, a cap (31) may further be placed onto the dispensing device, i.e. to suit and mask the dispensing device. Appropriate caps are those which comprise holes to let the vapor pass through the cap to the environment.

The heating element comprises any type of heating systems that would be available to the skilled person.

According to the instant invention, the heat element is powered by an autonomous system. The heating element does not require to be connected to a central electrical system to operate as would be any "plug-in" devices know in the art. Without being limitative, the power source may be any of a battery system, a solar cell system and the like. Other appropriate systems may be batteries which are regenerated by regular mean, typically standard AAA/AA battery charger, or by induction with a generator placed in a furniture such as a table.

By "heating system" it is understood an apparatus which generates heat to be transmitted to the volatile liquid contained into the intermediate basin. The thus generated heat forces the volatile composition to evaporate directly from the basin to the surrounding areas. This heating is discussed in the present description of the invention as an "active" release of the fragrance in opposition to the "passive" diffusion which corresponds to the release of the composition by convection from the dispensing material.

Examples of appropriate heating element correspond to light bulbs or any type of resistors.

According to one embodiment of the invention, at least one LED (light emitting diode) is present on the upper side (24) of the basin (2) in order to simulate candle flickering light. The LED is lighted during the heating period of the device and turns off when the period is over.

In reference to the embodiment illustrated in Figures 3 and 4, the heating element (4) and the power system (5) is located under the intermediate basin to directly heat the liquid contained in the intermediate basin (2), using a material for the basin which is suitable to transfer heat.

In reference to the embodiment illustrated in Figure 4, the different components of the dispensing device according to the invention are represented. First, a cap (31) may be placed onto the dispensing device; more precisely onto the basin (2) and the supply unit. Underneath the basin (2) is placed the heating element (4) which comprises any type of heating systems that could fit into the dispenser. The heating element (4) forces the volatile liquid to evaporate from the basin (2). Then, a battery case (5) may be placed underneath the heating element (4) in order to shelter the batteries necessary to operate the invention. Finally, a battery case lid (6) may be placed underneath the battery case (5) to protect the batteries and to close the battery case (5).

According to the invention, the heating element is activated cyclically, meaning throughout a series of substantially similar sequences reproduced at least twice over a given period of time and throughout the life time of the dispenser until it runs out of liquid in the reservoir and basin.

The dispenser according to the invention induces in addition to the continuous "passive" diffusion of the volatile liquid, the cyclic "active" release of the volatile liquid into the environment. When passive diffusion occurs, the consumers eventually gets used to the surrounding smell which limits the usefulness of the dispenser. By inducing actively a cyclic diffusion of the fragrance, this drawback can be overcome by breaking the habits of the consumers and ensuring that the concentration of volatile liquid into the atmosphere does not remain constant.

After intensive studies, it has been found that the heating may advantageously be performed cyclically each every 20 to 45 minutes, preferably each 30 minutes over a period of time which may vary from 10 seconds to 5 minutes, preferably 10 seconds to 2 minutes and more preferably 10 seconds to 1 minute.

The heating cycles of the device may be programmed to stop after the occurrence of a defined number of cycles, to be defined by the user.

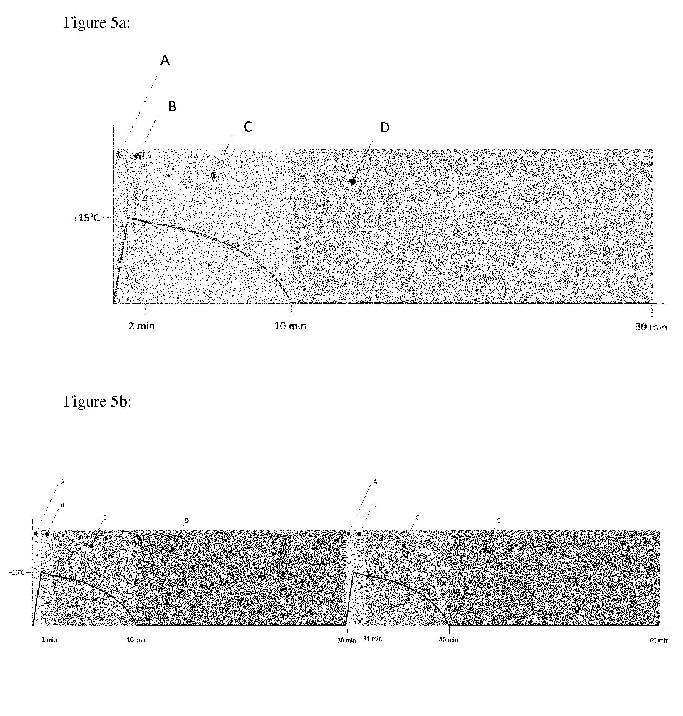

Typically, the heating element is set to raise the temperature of the volatile liquid by 20°C, preferably 15°C compared to the room temperature. The temperature of the volatile liquid is measured is the basin. During the heating period, the temperature can be either increased, linearly, randomly; maintained at a single temperature after a first increase in temperature or slowly ramped down to the initial temperature. In reference to the cycle depicted in Figure, 4, the heating period corresponds to the first 10 minutes, during which heating is performed over 30 seconds until the temperature raises by 15°C. At this point, smaller amount of energy is used to keep the temperature constant for an additional 30 seconds. The heating is then slowly decreased until the temperature returns to its initial value after 10 minutes, point at which no heating occurs for a period of 20 minutes. As depicted in Figure 5, another cycle is repeated after 30 minutes.

The Figure 5 represents a 2 hours sequence; with four heating cycles succeeding each other. In a first phase (A), the heating element is active at 100% and heats, for a period of 30 seconds, the liquid at a temperature up to 15°C above room temperature. In a second phase (B), the temperature is maintained at a temperature up to 15°C above room temperature for a period of 30 seconds, the heating element is active at 100%. In a third phase (C), the temperature is maintained for a period of 8 minutes; the heating element is active at 25%. In a fourth phase (D), there is no heating (the heating element is active at 0%) for a period of 20 minutes.

In addition to the cyclic activation of the heating, another embodiment of the dispenser according to the invention permits the user to force the activation of the heating on demand, regardless of the status of the cycle. This can be done for example by twisting or pushing an element of the dispenser which is connected to active the heating element.

Another aspect of the present invention is concerned with manufacture and the use of the supplying unit according to the invention.

When operating the dispensing device, the user, with a single step, places the unit with the opening section facing downwards onto a basin to open the reservoir, release the volatile liquid and dispense the volatile liquid within the surrounding atmosphere. This process has been advantageously simplified in comparison to the existing dispensers.

## Claims

1. An autonomously powered vapor dispensing device comprising
- an intermediate basin which allows the volatile liquid contained therein to impregnate a dispensing material by capillarity and dispense into the atmosphere;
- a heating element which heats the intermediate basin comprising the volatile liquid to force evaporation cyclically of the volatile liquid directly from the intermediate basin.

2. The dispensing device according to claim 1, wherein the intermediate basin is fed by a replaceable reservoir to be placed onto the intermediate basin and is closed by a rupturable substrate that opens upon placing on said basin.

3. The dispensing device according to any of claims 1 or 2, wherein the dispersing material is framed with a coupling element which holds together the reservoir and the dispensing material onto the basin, preferably in a vertically straight position.

4. The dispensing device according to any of claims 1 to 3, wherein the level of volatile liquid contained into the basin is maintained constant throughout the regular use of the device.

5. An autonomously powered vapor dispensing device adapted to release a volatile liquid from an impregnated dispensing material by passive convection and further adapted to cyclically heat the volatile liquid over a period of 10 seconds to 5 minutes, preferably 10 seconds to 2 minutes, more preferably 10 seconds to 1 minute to enhance said convection.

6. The dispensing device according to claim 5, wherein the cycles are repeated each 20 to 45 minutes, preferably each 30 minutes.

7. The dispensing device according to anyone of claims 5 or 6, wherein the device is programmed to automatically stop after the occurrence of a preset number of cycles.

8. The dispensing device according to anyone of claims 5 to 7, wherein the temperature of the volatile liquid is heated by 15°C, preferably by 10°C and more preferably by 5°C above room temperature.

9. The dispensing device according to claim 8, wherein a constant temperature above room temperature is constantly maintained over a predetermined period of time of more than 10 seconds and does not exceed 7 minutes, preferably does not exceed 4 minutes and more preferably does not exceed 2 minutes.

10. The dispensing device according to anyone of claims 1 to 9 which is powered by batteries.

11. The dispensing device according to claim 10 wherein the batteries are rechargeable by regular mean or by induction.

12. The dispensing device according to claim 1 to 11 wherein the heating element corresponds to at least one light bulb or at least one resistor.

13. Process for the manufacture of the device according to anyone of claims 1 to 4, comprising the steps consisting in fixing a reservoir in the neck of the dispensing device dispensing in order to release the volatile liquid into the intermediate basin.

14. Use of a dispensing device according to anyone of claims 1 to 12 to release a fragrance.

15. Use according to claim 14 which further prevents the habituation of the fragrance to the user over the entire lifetime of the dispenser.
